(19) Europäisches Patentamt
European Patent Office
Office européen des brevets

(11) Publication number: 0 290 102 A2

# (12) EUROPEAN PATENT APPLICATION

(21) Application number: 88200884.0

(22) Date of filing: 04.05.88

(51) Int. Cl.4: C07D 251/08 , C07D 403/04 , C07D 405/04 , C07D 473/00 , C05C 9/02 , C05C 9/00

(30) Priority: 07.05.87 US 46647

(43) Date of publication of application: 09.11.88 Bulletin 88/45

(84) Designated Contracting States: BE DE ES FR GB IT NL

(71) Applicant: ARCADIAN CORPORATION
1 Gatehall Center
Parsappany New Jersey(US)

(72) Inventor: Hawkins, Edwin Francis
1238 Stoneleigh Drive
Baton Rouge Louisiana(US)
Inventor: Clapp, John Garland
301 Clapp Farms Road
Greensboro, N.C. 27405(US)

(74) Representative: van der Saag, Johannes et al
OCTROOIBUREAU VRIESENDORP & GAADE
P.O. Box 266
NL-2501 AW The Hague(NL)

## (54) Water-insoluble triazone fertilizer and method of making and use.

(57) In a preferred embodiment, a high-yield method producing novel water insoluble triazone compositions with a typical analysis prior to separation of the insoluble crystals, percentages based on total weight of reaction produced, includes insoluble triazone at about 75 percent, produced by admixing urea with formaldehyde while adjusting and maintaining pH at about pH 9, and while maintaining well mixed, heating for about one hour at about 85°C in water solvent, followed by adding ammonia while maintaining temperature below about 65° and pH at about pH 9 for about an hour, followed by cooling to about 30°C, filtering-off insoluble triazone crystals, washing the crystals and drying the crystals. Novel fertilizer-type insoluble triazones typically include methyl triazone and hydroxyethyl triazone and hydroxyethyl thiotriazone, applicable to land or crops alone or in combination with other fertilizers.

EP 0 290 102 A2

# WATER-INSOLUBLE TRIAZONE FERTILIZER AND METHOD OF MAKING AND USE

This invention is directed to a novel method for producing water-insoluble triazones for use thereof as water-insoluble fertilizer(s).

## BACKGROUND TO THE INVENTION

Prior to this invention, the present joint inventor Hawkins was granted United States patents nos. 4 544 005 granted November 19, 1985 and 4 599 102 granted July 8, 1986 respectively, directed to water-soluble triazone compositions and their methods of preparation and use as foliar fertilizers. It is assumed for purposes of this patent application that a material is considered to be water-soluble if a solution, containing at least eight percent or more by weight of nitrogen can be prepared at standard temperature and pressure, and likewise is considered to be water-insoluble if less than eight percent water soluble nitrogen solution can be prepared from the substances.

It was desirable to obtain novel water-insoluble triazone(s), of which it was not at all clear whether or not many particular insoluble triazone compounds could or could not be produced, or if so, whether in any significant and practical yield, as well as many insoluble triazones never having existed before. Just as with the water-soluble triazones, it is not possible to reliably predict whether it will have beneficial fertilizer utility or alternatively will adversely affect or kill vegetation, as opposed to helping it. Such can be ascertained solely by field "trial and error", toxicity to plant life not being predictable.

It has also been desirable to substantially increase the yield of the water insoluble triazone compositions to a significant degree such that they may be economically and practically produced on a commercial scale, by novel method(s) of this invention.

## OBJECTS OF THE INVENTION

Accordingly, objects of the present invention include the obtaining of novel water-insoluble compositions and a novel method obtaining unexpectedly high yields of water-insoluble triazone(s) in the reaction product as compared to what was known or possible heretofore, and new use of particular water-insoluble triazones, including some novel water-insoluble compounds as fertilizers, particularly as applied to or into the soil.

Another object is to ascertain whether there exist particular ranges of various ingredients to obtain unexpectedly improved yields of water-insoluble triazones utilizable as fertilizers.

Another object is to ascertain whether among apparent equivalents for reactants, and particular ranges obtain unexpectedly improved yields of water-insoluble triazones.

## SUMMARY OF THE INVENTION

Broadly, the invention may be described as a novel method obtaining unexpectedly high field of water-insoluble triazone compositions of the above-noted patents and of producing novel water-insoluble triazone compositions noted below, and novel use of particular water-insoluble triazone compositions as fertilizers.

It was unexpectedly discovered that superior results could be achieved by a first method having improved yields of insoluble triazone, and a second method especially high yields of the water-insoluble thiotriazones, more particularly made in even higher yields, more preferably by utilizing repeatedly the mother liquid, remaining after separation of the insoluble triazone crystals.

More, particularly, the first method admixes a urea-type compound-source, such as formaldehyde, and a diluent, such as water, and/or another diluent for dissolving and/or suspending the reactants, using the diluent in a diluting amount sufficient for the foregoing reactants to form and exist as a reaction mixture. These reactants are thereupon mixed to form the reaction mixture (and/or solution), to which there is added a caustic in an amount sufficient to adjust pH within a range of about pH 8.6 to about pH 9.3, and sufficiently to thereby form a pH-adjusted reaction mixture. Thereafter, to the pH-adjusted reaction mixture there is added an ammonia-type compound-source, such as ammonia and/or amine and/or substituted

amine; it/they is/are added in an amount sufficient to form an ammonia or amine type reaction product. When the ammonia-type compound-source includes an amine (or substituted amine), the rate of addition of the ammonia type compound source is added at a sufficiently slow rate of addition, relative to the surrounding natural or implemented cooling-conditions, as to maintain temperature up to a temperature below a boiling point of the amine (or substituted amine), relative to rate of cooling of the ammonia and/or amine type reaction product. Thereafter, the resulting ammonia, amine or substituted-amine-type reaction product is heated at a temperature within a temperature range of from about 85°C to about 93°C for an initial heating period product mixture while again (a second time) adding caustic in an additional amount, sufficiently to maintain pH within the above-described pH range (described for the initial heating period). Thereafter, further addition of caustic is terminated, while thereafter continuing heating the final reaction product mixture within the temperature range above-described pH range (described for the initial heating period). Thereafter, further addition of caustic is terminated, which thereafter continuing heating the final reaction product mixture within the temperature range above-described for the initial heating, for an additional heating period ranging from about 5 minutes to about 25 minutes, sufficiently to form a cooked product mixture. Thereafter, the cooked product mixture is cooled sufficiently such that insoluble triazone becomes substantially crystallized triazone apart from remaining liquid product and substances dissolved therein. Thereafter, the crystallized triazone is separated from the remaining mother liquor and products dissolved therein, by any of desired and/or conventional routes/methods that are not detrimental to the crystallized triazone. Typically, centrification and/or filtration may be employed, for example.

In this first method, preferably the diluent includes a major proportion of water, possibly solely water.

In a preferred embodiment of this first method, the temperatures of reaction as described-above for this fourth method, are maintained within a temperature range of from about 89 to about 91°C.

In this first method, preferably upon the first-adding and the second adding of caustic, the caustic is added in amounts such that pH-alkalines range between about pH 8.9 and pH 9.1.

In a preferred embodiment of the first method, initial heating ranges from about 15 to about 25 minutes, and the additional subsequent heating ranges from about 7 minutes to about 15 minutes.

In this first method, preferably the cooling includes cooling-down the cooked-product mixture to at least below about 30°C.

In the first method, preferably the urea-type compound is at least predominantly urea, and the aldehyde type compound-source includes at least predominantly formaldehyde, and the caustic is at least predominantly potassium hydroxide.

In this first method, preferably after the separating of the insoluble crystals, there is included drying the separated crystals of insoluble triazone at a temperature of from about 60°C to about 75°C for at least about 36 hours.

Also in the foregoing first method, preferably in the cooling prior to the separating, that cooling includes the cooling of the cooked product for a final cooling period of at least about 60 hours at a temperature of between about 0.5°C and about 2°C.

Additionally, in the first method, preferably after the separating of the crystals, they are dried at a temperature of from about 65°C to about 72°C for at least about 44 hours.

A second novel especially effective method is utilized most effectively and favourably in the production of various thiotriazones. Initial reactants include (and are normally and preferably limited to) urea-type compound source(s), such as urea and/or substituted urea and/or mixture(s) thereof with aldehyde (or substituted aldehyde) and an aldehyde-type compound-source, such as formaldehyde and/or actealdehyde and/or substituted forms thereof, and/or the like, and a diluent, such as water and/or other diluent(s), in an amount sufficient to form a reaction mixture (and/or solution) of reactants. These initial reactants are thereupon admixed sufficiently to form a reaction mixture (such term intended to include mixture and/or solution) to the reaction mixture, there is thereafter added ammonia and/or amine type and/or substituted amine type compound, in amount(s) sufficiently to form an ammonia or amine type product. When the ammonia type compound is or included an amine and/or substituted amine, rate of addition of the ammonia type compound is a rate sufficiently slow during the addition, as to maintain temperature up to a temperature below a boiling point of the amine(s) and/or substituted-amines, relative to normal cooling conditions and/or initiated cooling conditions to which the mixture is subjected, relative to rate of cooling of the ammonia and/or amine(s) and/or substituted-amines being added. Thereafter, the thereby formed ammonia and/or amine and/or substituted amine type product(s) are cooled sufficiently that insoluble triazone(s) become substantially crystallized as crystallized triazone(s) apart from remaining mother liquor product and substance(s) dissolved therein. Thereafter, the insoluble crystallized triazone(s) is/are separated from remaining liquid products and substances dissolved therein.

In the foregoing second method, preferably the initial mixing is continued for a period of at least about

15 minutes.

In the second method, preferably the diluent includes a major proportion of water.

In the second method, preferably the aldehyde type compound includes at least predominantly formaldehyde.

In the second method, preferably the cooling, prior to the separating, includes cooling the ammonia and/or amine type and/or substituted amine type product for a final cooling-period of at least 48 hours at a temperature between about 0.2° and about 6°C.

In the second method, preferably after separating, the crystallized triazones are dried at a temperature of from about 60° to about 75°C for at least about 36 hours.

In this second method, also preferably, during the cooling prior to separating of the insoluble crystals of triazone, the cooked product is cooled for a final cooling period of at least about 60 hours at a temperature of between about 0.5° and about 2°C.

Preferably, after separating the insoluble crystals of triazone from the mother liquor, the crystals are dried at a temperature of from about 60° to about 75°C for at least 36 hours.

Also, preferably in the second method, prior to separating the insoluble crystals from the mother liquor, the cooked product is cooled for a final cooling period of at least 60 hours at a temperature of between about 0.5° and about 2°C.

After separating the insoluble crystals, preferably they are dried at a temperature of from about 65° to about 72°C for at least about 44 hours.

For all the reactions of the various methods, unless otherwise specified, the molar ratios are as follow:

For monoamines such as methyl amine, ethyl amine, ethanol amine, and the like:
Urea/HCO/amine at 1/2/1

for diamines such as ethylene diamine:
urea/HCHO/amine at 3/6/1

i.e. for complete reaction, one mole of urea (for example) and two moles of formaldehyde (for example) are needed for each primary amine group in the amine molecule.

A small excess of urea and formaldehyde may be desirable to insure that all of the amine is reacted.

In all of the above formulations, typical substitutions may be made as follows: for urea, there may be substituted in whole or in part thiourea, dimethyl urea, ethyl urea and the like. For formaldehyde, there may be substituted in whole or in part acetaldehyde, propionaldehyde and the like.

It is to be noted that even when a novel insoluble trizone produced by the methods of this invention, such as above-noted compounds, in some instances lack utility as a fertilizer because of phytotoxicity or other detriment to plant life, that particular triazone nevertheless has a utility as a weed killer or defoliant or growth regulator or the like, i.e. a different or opposite utility.

It was not possible to reliably predict that these compounds could be produced nor that if produced, that they would be insoluble and stable as to shelf life and at varying storage temperatures and periods of time.

As a further practice of increasing the yields of the solid products of the mother liquor from one crystallization may be utilized as the diluent to product subsequent batches of the same water insoluble triazone.

Second, the mother liquor itself contains nitrogen from the small amount of unreacted urea and from the dissolved (soluble) triazones therein, and with adjustment may be used as a nitrogen source for fertilizer thus substantially eliminating any problem of disposal of waste products.

Particular insoluble triazones having required acceptable fertilizer properties and characteristics within the scope of this invention, include the following.

Methyl triazone or ethyl triazone - where the methyl or ethyl may be at any of alternate carbon-substituted positions or at a free nitrogen position (as 5-methyl, for example), such as;
methyl triazone produced from urea (or equivalent), methyl amine, and formaldehyde (or equivalent)
5-B hydroxyethyltriazone
5-(furfuryl) triazone
5 purine triazone
ditriazone entitled isopropyl ditriazone and ethylene ditriazone
thiotriazone - and alkyl carbon substituted and/or alkanol N-substituted forms thereof, such as typically 1-3

dimethyl thiotriazone, produceable from 1,3 dimethyl thiourea, ammonia and formaldehyde; and typically 1-3 dimethyl-5-Beta ethanol thiotriazone; and typically 4-6 dimethyl thiotriazone; and typically 1,3,4,6 tetramethyl thiotriazone; and typically 1,3,4,5,6 pentamethyl thiotriazone. dithiotriazones and alkyl carbon-substituted forms thereof, such as 5,5' ethylene dithiotriazone of the structural formula:

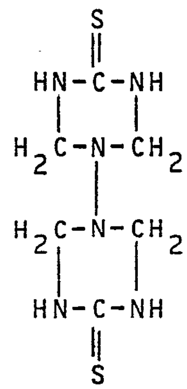

1,3,5-trimethyl thiotriazone
4,6,5-trimethyl thiotriazone
4,6-diethyl thiotriazone
4.6-diethyl-5-B hydroxyethyl thiozone
1,3-dimethyl thiotriazone, produceable from 1,3-dimethyl thiourea, ammonia and formaldehyde
4,6-dimethyl thiotriazone
4,6-propyl-5-betahydroxyethyl triazone($C_{11}H_{24}N_3O_2$)
that has a molecular weight of 230 (18.3%);produced from urea, isobutyraldehyde and B-ethanol-amine.
ethyl triazone produced from urea (or equivalent), ammonia and formaldehyde (or other aldehyde equivalent) isopropyl ditriazone of the structural formula:

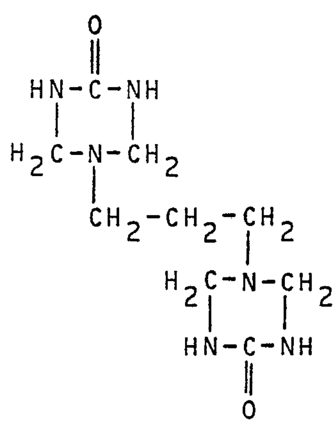

5-hydantoinamid, triazone
1, 4 triazone, 2 hydroxypropane
methyl thiotriazone
ethanol thiotriazone
5, 5' ethylene dithiotriazone
1,3,5 triamethyl thiotriazone
4, 6, 5 trimethyl thiotriazone
4,6 diethyl thiotriazone
4,6 diethyl thiotriazone
4,6 diethyl-5-β-hydroxyethyl thiotriazone
ethyl thiotriazone
S-adamantanetriazone ($C_{13}H_{21}N_3O$)
having a molecular weight of 235 (17.9% N) produced from urea, formaldehyde and adantanamine

5-(5, 6-dimethyl-1, 2,4-triazine) triazone ($C_8H_{12}N_6O$) having a molecular weight of 208 (40% N); produced from urea,
formaldehyde and 3-amino-5, 6-dimethyl-1, 2, 4 trizine.
5-(1 3 dimethyl uracil) triazone ($C_9N_{15}N_5O_3$) and the like

Novel insoluble thiotriazones generically include at least the following

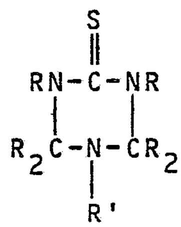

where R is hydrogen, alkyl (such as methyl, ethyl, dimethyl, trimethyl, etc.) alkyl-substituted alkyl, alkylamine and/or cycloalkyl, and where R′ is hydrogen, alkyl or alkyl-substituted alkyl, or alkylene, or cycloalkyl or alkyl-amine.

The typical approximate yields of various products prepared by the preceding preferred methods, in terms of percentage yields based on amine addition, for crude product (not crystallized), are ethylene ditriazone at 47%, methyl thiotriazone at 76%, hydroxyethyl thiotriazine at 40%, methyl triazone at 60%, thiotriazone at 42%, and hydroxyethyl triazone at 35%.

## DETAILED DESCRIPTION

As a typical illustration that not all random methods may be utilized to produce the insoluble triazone of this invention, the following Example I is an actual experiment that did not work, i.e. was an unsuccessful procedure.

### Example I

Example I

| Reactants-formulation: | Wt.% | grams |
|---|---|---|
| A) Thiourea | 31.1 | 1244 |
| B) Paraformaldehyde | 24.5 | 1380 |
| C) Anhydrous ammonia | 7.0 | 280 |
| D) Water | 37.4 | 1496 |

Results: A gummy white insoluble precipitate formed. It was very difficult to filter or clean-up. Upon storing in the refrigerator overnight at 35°C, no further precipitate, except the water insoluble gum. The gum product had no apparent utility.

### Example II

Methyl triazone alone or usually also hydroxyethyl triazone were evaluated at several different research locations including comparisons thereof with:

a) separately liquid urea and URA et La Belle, Florida, on sod Floratane St. Augustine and 419 Hybrid Bermuda; and

b) urea, on blugrass, at Allentown, NJ; and

c) separately urea and ammonium nitrate, on turf grass, tall fescue; and

d) separately urea and IBDU, with regard to mineralization and volatalization, at Kansas State University.

In all instances, the compound methyl triazone and/or hydroxyethyl triazone obtained superior results, re color ratings indicative of lack of phytotoxicity-leaf burn or injury, and superior growth ratings, and significantly lower mineralization at 22°C and 32°C, and significantly lower volatilization at 22°C. Volatilization from surface-applied urea can be over 50 percent, a major problem.

## Example III

This example was directed to and is typical of one of many experiments, in which water-insoluble triazone was produced, here producing methyl triazone.

| Formulation | Wt.% |
|---|---|
| UF-85 (BORDEN/26.6% urea & 57.7% formaldehyde) | 31.2 |
| Urea | 49.2 |
| 25% KOH-solution | 3.6 |
| Methylamine (40% solution) | 16.0 |

Procedure followed:

A. Mixed together the UF-84 and urea.

B. Slowly added the methylamine solution.

C. Heated to 90°C (plus or minus 0.5°) for one hour while adjusting pH at about 9.0 (plus or minus about 0.3) for one hour by slow and gradual addition of KOH solution.

D. Maintained temperature at about 90°C (plus or minus 0.5°) for an additional hour.

E. Cooled and packaged. Heavy crystallization on standing 24 hours, occurred. Crystals were filtered off, and washed with reagent alcohol, and again crystals were filtered off. Filtered crystals were dried at 50°C in a vacuum oven.

## Example IV

In another experiment the following procedure was followed.

1) There were mixed together urea and urea-formaldehyde mixture (commercial UF-85), and water.

2) Adjust pH to 9.0 with 45% KOH aqueous solution (a typical standard laboratory dilution).

3) Slowly add the amine (and/or ammonia) while cooling to the extent required to keep the temperature below the boiling point of the amine. (The boiling point of the amines vary from about 45° to over 100°, depending upon which amine is utilized. One must not allow the temperature to get above about 85°C in any case during the amine addition).

4) Heat to 90°C and maintain temperature at 90° (plus or minus about 1°C) for about 20 minutes while holding pH at about 9.0 (plus or minus about -.3) by the slow addition of 45 percent KOH solution.

5) End pH control but continue temperature control at 90°C (plus or minus about 1°C) for an additional about ten minutes.

6) Cool to room temperature.

7) Transfer the reaction mix to a 4 liter beaker and cool for about 48 to 72 hours at 1°C (34°F). In some of the products, crystallization occurs during the amine addition and cooling lower than room temperature is not required.

8) Filter-off the crystals and wash with reagent alcohol twice, or with water. Crystals may be removed from the mother liquor by filtration, centrifuging, or sedimentation or any other desired method that would not be detrimental to the crystals.

Products produced by this last described method are as follows: 5-B, hydroxyethyl triazone; 5-methyl triazone; 5-ethyl triazone; ethylene ditriazone; isopropyl ditriazone; 1, 3 ditriazone, 2 hydroxypropane; tris (ethyl triazone) amine; S-adamatane triazone; 5 purine triazone; 1, triazone, 3, (2, 5 dioxo-4-imidazolidinyl urea; 5-(1,3 dimethyl uracil) triazone; furfurylamine.

Example V

This example follows a typical procedure of the other thus far optimal method researched and developed, for producing insoluble triazone, but found best suited for, i.e. best results in, production of thiotriazones, according to the following typical procedure followed in the laboratory.

1) Mix together the urea (or substituted urea), the urea-formaldehyde mixture (i.e. typically a mixture of commerically available mixture of urea and formaldehyde known as UF-85), and water (and/or other diluent).

2) Slowly add the amine (and/or ammonia) while cooling sufficiently as might be required to keep the temperature below the boiling point of the amine. The boiling point of the amines varies for different amines from about 45°C to about (or more than) 100°C. Do not allow the temperature to get above about 85°C in situations utilizing the amine addition.

3) Allow mixing to continue for at least about 30 minutes. Preferably mix for at least about 15 minutes up to about 3 hours.

4. Cool to about room temperature.

5. Transfer the reaction mix to a 4 liter beaker and cool for about 48 to about 72 hours at about 1°C (34°F). In some of the products, crystallization occurs during the amine addition and cooling lower than room temperature is not required.

6) Filter off the crystals and wash with reagent alcohol (or water) twice. Crystals may be removed from the mother liquor by filtration, centrifuging, sedimentation or any desired method.

7). Oven dry the crystals at about 70°C from about 48 hours. Product may be spray dried, freeze dried or oven dried, or dried by any other desired applicable method that does not damage the crystals.

Typical insoluble thiotriazones optimally produced by the last described above method of Example IX include the following:
unsubstituted thiotriazone; 5-B-ethanol thiotriazone; 5-ethyl thiotriazone; 5,5′-ethylene dithiotriazone; and 4,6-diethyl, 5-B-hydroxyethyl thiotriazone.

It is within the scope of this invention to make variation and substitution of equivalents obvious to a person of ordinary skill in this art.

## Claims

1. A method of making a water-insoluble triazone comprising 1) employing initial reactants comprising urea-type compound-source and an aldehyde-type compound-source and a diluent in a diluting amount sufficient for the urea-type compound source to be admixed therein sufficiently to form a reaction mixture; 2) mixing-together said initial reactants sufficient to obtain said reaction mixture; 3) first-adding caustic in an amount sufficient to adjust pH to a pH within pH range from a out pH 8.6 to pH 9.3, and sufficiently to form a pH-adjusted reaction mixture; 4) thereafter to said pH-adjusted reaction mixture, adding an ammonia-type compound-source sufficiently to form an ammonia or amine-type reaction product, when said ammonia-type compound-source includes an amine, rate of addition of said ammonia-type compound-source being sufficiently slow during said adding as to maintain temperature up to a temperature below a boiling point of said amine, relative to rate of cooling of the ammonia or amine-type reaction product; 5) thereafter heating said amine-type reaction product at a temperature within a temperature ranging from about 85°C to about 93°C for an initial heating period ranging from about 15 minutes up to about 60 minutes sufficiently to form a final reaction product mixture, and concurrently second-adding caustic if necessary in an amount sufficient to substantially maintain pH within said pH range during said initial heating period; 6) thereafter terminating further addition of caustic, and concurrently substantially maintaining temperature of said final reaction product mixture within said temperature range for an additional period ranging from about 5

minutes up to about 60 minutes, sufficiently to form a cooked product mixture; 7) thereafter cooling said cooked product mixture sufficiently that insoluble triazone becomes substantially crystallized triazone apart from remaining liquid product and substances dissolved therein; and thereafter separating said crystallized triazone from said remaining liquid product and substances dissolved therein.

2. The method of claim 1, in which said diluent includes a major proportion of water, and in which said temperatures of said temperature range is maintained within a second temperature range of from about 98 to about 91°C, in which said first-adding and said second-adding of caustic is in an amount such that pH-alkalinities of said pH range is maintained between about pH 8.9 and pH 9.1, heating during said initial heating ranging from about 15 to about 25 minutes, and said additional heating ranging from about 7 minutes to about 15 minutes, and in which said cooling comprising cooling-down said cooked-product mixture to at least below about 30°C.

3. The method of claim 2, in which said urea-type compound source is at least predominantly urea, and in which said aldehyde-type compound source comprises at least predominantly formaldehyde, and said caustic is at least predominantly potassium hydroxide.

4. The method of claim 3, in which said cooling, prior to said separating, comprises cooling said cooked product for a final cooling-period of at least 48 hours at a temperature of between about 0.2° and 6°C.

5. The method of claim 4, including, after said separating, drying said crystalized triazone at a temperature of from about 60° to about 75°C for at least about 36 hours.

6. The method of claim 3, in which said cooling, prior to said separating, comprises cooling said cooked product for a final cooling period of at least about 60 hours at a temperature of between about 0.5° and about 2°C, and after said separating, drying said crystallized triazone at a temperature of from about 65° to about 72°C for at least about 44 hours.

7. A method of making a water-insoluble triazone comprising 1) employing initial reactants comprising a urea-type compound-source and an aldehyde-type compound-source and a diluent in a diluting amount sufficient for the urea-type compound source and the aldehyde-type compound-source to be admixed therein sufficiently to form a reaction mixture; 2) mixing-together said initial reactants sufficiently to form said reaction mixture; 3) thereafter adding an ammonia-type compound-source sufficiently to form an ammonia or amine-type reaction product sufficiently to form an ammonia or amine-type product, and 4) when said ammonia-type compound-source includes an amine, rate of addition of said ammonia-type compound-source being sufficiently slow during said adding as to maintain temperature up to a temperature below a boiling point of said amine, relative to rate of cooling of said ammonia or amine-type compound-source; 5) thereafter cooling said ammonia or amine-type product sufficiently that insoluble triazone becomes substantially crystallized as crystallized triazone apart from remaining liquid product and substances dissolved therein; and 6) thereafter separating said crystallized triazone from said remaining liquid product and substances dissolved therein.

8. The method of claim 7, in which said mixing is continued for a period of at least about 15 minutes, in which said diluent includes a major proportion of water, and in which said cooling comprises cooling-down said ammonia or amine-type product to at least below about 30°C.

9. The method of claim 8, in which said urea-type compound comprises at least predominantly a thiourea, and in which said aldehyde-type compound comprises at least predominantly formaldehyde.

10. The method of claim 9, in which said cooling, prior to said separating, comprises cooling said ammonia or amine-type product for a final cooling period of at least about 48 hours at a temperature between about 0.2° and 6°C.

11. The method of claim 10, including, after said separating, drying said crystallized triazone at a temperature of from about 60° to about 75°C for at least about 36 hours.

12. The method of claim 10, in which said cooling, prior to said separating, comprises cooling said cooked product for a final cooling period of at least about 60 hours at a temperature of between about 0.5° and about 2°C, and after said separating, drying said crystallized triazone at a temperature of from about 65° to about 72°C for at least about 44 hours.

13. A water-insoluble triazone entitled 5-B, hydroxethyltriazone.

14. A water-insoluble triazone entitled 5,5′-ethylene ditriazone.

15. A water-insoluble triazone entitled 1,3-ditriazone, 2 hydroxypropane.

16. A water-insoluble triazone entitled methyl thiotriazone.

17. A water-insoluble triazone entitled ethyl thiotriazone.

18. A water-insoluble triaonze entitled ethyl thiotriazone.

19. A water-insoluble triazone entitled ethanol thiotriazone.

20. A water-insoluble thiotriazone of the structural formula:

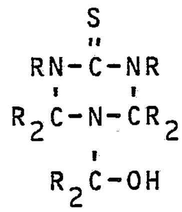

in which R is hydrogen, alhky, and/or alkyl-substitute alkyl, and in which R′is alkyl, alkyl-substituted alkyl, or alkylene.

21. A water-insoluble thiotriazone of the structural formula:

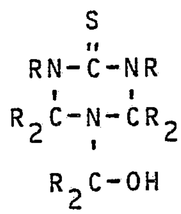

where R is hydrogen, alkyl, and/or alkyl-substituted alkyl.

22. A water-insoluble thiotriazone entitled thiotriazone of the structural formula:

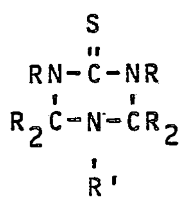

in which R is hydrogen, alhyl or alhyl-substituted-alkyl, and where R′ is alkyhl or alkyl-substituted alkyl, cycloalkyl, hydroxyalkyl, or alkylene.

23. A water-insoluble thiotriazone entitled 1-3 dimethyl thiotriazone.

24. A water-insoluble thiotriazone entitled 1-3 dimethyl-5-Beta ethanol thiotriazone.

25. A water-insoluble thiotriazone entitled 1, 3, 4, 6-tetramethyl thiotriazone.

26. A water-insoluble triazone entitled 5(furfuryl) triazone.

27. A water-insoluble triazone entitled 5-hydrantoinamid, triazone.

28. A water insoluble triazone entitled 5 purine triazone.

29. A water-insoluble triazone entitled ethyl triazone.

30. A water-insoluble triazone entitled isopropyl ditriazone.

31. A water-insoluble triazone entitled ethylene ditriazone.

32. A water-insoluble thiotriazone entitled 1-3-5-trimethyl thiotriazone.

33. A water-insoluble thiotriazone entitled 4, 6, 5, trimethyl thiotriazone.